# FASCICULE DE BREVET EUROPEEN

(11) **EP 0 609 124 B1**
(45) Date de publication et mention de la délivrance du brevet: **31.07.1996**
(21) Numéro de dépôt: 94400140.3
(22) Date de dépôt: 24.01.1994
(51) Int. Cl.: C07C 17/20, C07C 19/08

(54) **Procédé de fabrication du 1,1,1,2-tetrafluoro-2-chloroethane et du pentafluoroethane**
Verfahren zur Herstellung von 1,1,1,2-Tetrafluor-2-Chlorethan und Pentafluorethan
Process for the preparation of 1,1,1,2-tetrafluoro-2-chloroethan and pentafluorethane

(30) Priorité: 27.01.1993 FR 9300780
(43) Date de publication de la demande: 03.08.1994
(73) Titulaire: ELF ATOCHEM S.A., F-92800 Puteaux (FR)
(72) Inventeur: Cheminal, Bernard, F-69510 Soucieu-en-Jarrest (FR); Lacroix, Eric, F-69480 Amberieux D'Azergues (FR); Lantz, André, F-69390 Vernaison (FR)
(74) Mandataire: Leboulenger, Jean

(56) Documents cités:
- EP-A- 0 328 127
- EP-A- 0 349 298
- EP-A- 0 486 333
- WO-A-92/16482

## Description

La présente invention concerne un procédé continu de fabrication du 1,1,1,2-tétrafluoro-2-chloroéthane (F124) et du pentafluoroéthane (F125) et a plus particulièrement pour objet la fabrication de ces deux composés par fluoration en phase gazeuse du 1,1,1-trifluoro-2,2-dichloroéthane (F123) au moyen d'acide fluorhydrique en présence d'un catalyseur.

Les composés F124 et F125 pouvant être utilisés comme substituts des perchlorofluorocarbures (CFC) dans le domaine des aérosols (agents propulseurs) et dans celui de la réfrigération, on recherche actuellement des procédés performants pour leur production industrielle.

Dans le brevet US 4 766 260 est décrit un procédé de synthèse des composés F123 et F124 par hydrofluoration d'oléfines perhalogénées en phase gazeuse, l'objectif étant de minimiser la formation de F125. L'exemple 13 (colonne 6) décrit la fluoration du tétrachloroéthylène avec un catalyseur CrCl₃/Al₂O₃; malgré une température de 350°C, un long temps de contact (60 secondes) et un rapport molaire HF/C₂Cl₄ élevé (6/1), les sélectivités en F124 et F125 sont faibles (33,3 % et 7,2 % respectivement).

L'emploi d'un catalyseur à base de chrome III (CrCl₃) supporté sur charbon pour la fluoration catalytique en phase gazeuse d'oléfines halogénées fait l'objet de la demande de brevet japonaise publiée sous le n° 48-72105/73 dont l'exemple 4 décrit la fluoration du tétrachloroéthylène. Là encore, malgré une température de réaction de 400°C et un rapport molaire HF/C₂Cl₄ élevé (5/1), la composition des produits formés se limite au F121 (CHCl₂-CFCl₂: 6,8 %), au F122 (CHCl₂-CClF₂: 10,5%) et au F123 (82,7%).

Le brevet US 3 258 500 décrit l'utilisation de chrome massique ou supporté sur alumine pour des réactions de fluoration catalytique en phase gazeuse. En particulier, l'exemple 17 (colonne 14) décrit la fluoration du tétrachloroéthylène. A 400°C avec un rapport molaire HF/C₂Cl₄ de 6,2/1, la sélectivité en F123 + F124 + F125 est faible (47,7 %); une diminution de la température de réaction (300°C) améliore cette sélectivité (79,7 %) mais la distribution est alors déplacée vers les produits moins fluorés (F123 et F124).

La demande de brevet EP 0 349 298 décrit la synthèse des composés F123 et F124 à partir de pentahalogénoéthanes par fluoration catalytique en phase gazeuse sur un catalyseur composé d'un métal choisi parmi le chrome, le cobalt, le nickel et le manganèse, déposé sur alumine. Ce document met l'accent, d'une part, sur l'activation à l'acide fluorhydrique poussée du catalyseur (au moins 90 % du support sous forme de AlF₃ après activation) et, d'autre part, la formation minimisée de F125 durant la réaction. Ainsi, dans l'exemple 6 qui décrit la fluoration du F122 à 350°C et avec un long temps de contact (30 secondes), la sélectivité en F125 n'est que de 1,1 % et la sélectivité cumulée (F123 + F124 + F125) est seulement de 71,5 %. Dans l'exemple 5 qui décrit la fluoration du F123 en phase gazeuse sur un catalyseur NiCl₂/Al₂O₃ à 400°C, avec un temps de contact de 30 secondes et un rapport molaire HF/F123 de 4, la sélectivité en F125 est seulement de 7,5 %.

Un procédé pour produire des hydrocarbures aliphatiques fluorés, basé sur la fluoration par l'acide fluorhydrique en phase gazeuse d'hydrocarbures aliphatiques halogénés contenant au moins un atome d'halogène autre que le fluor, fait l'objet du brevet US 3 755 477 où le catalyseur est un oxyde chrome massique traité à la vapeur avant calcination et activation à l'acide fluorhydrique. L'exemple 25 utilise un tel catalyseur pour la fluoration du F123 à 390°C avec un rapport molaire HF/F123 élevé (9,5/1); les sélectivités en F125 et F124 sont respectivement de 67 et 21 %, mais on note aussi une sélectivité de 2,5 % en chloropentafluoroéthane (F115) non recyclable.

De l'examen de l'état de la technique, il apparaît difficile de synthétiser les deux composés désirés (F124 et F125) avec une bonne sélectivité et une productivité importante par fluoration directe du tétrachloroéthylène ou du F122. Au départ du tétrachloroéthylène et malgré des temps de contact, des températures et des rapports molaires élevés, il semble difficile d'obtenir le F124 et plus spécialement le F125 avec de bons rendements. La synthèse de ces deux composés est plus facile à partir du F122, mais on est confronté dans ce cas à un problème de sélectivité (formation importante de sous-produits).

Quant au brevet US 3 755 477, il montre qu'à partir du F123 la synthèse du F125 requiert un rapport molaire (9,5) et une température (390°C) élevés conduisant concomitamment à la formation non négligeable de F115 indésirable.

La demande de brevet WO 92/16482 concerne la fabrication du F124 et du F125 par fluoration en phase gazeuse de pentahalogénoéthanes, notamment celle du F123, sur un catalyseur à base de zinc et éventuellement d'un autre métal déposé(s) sur une alumine fluorée. Malgré un temps de contact élevé (30 secondes), le meilleur taux de transformation du F123 obtenu dans les exemples est d'environ 60 % et la sélectivité maximale en F125 est de 28 %.

La demande de brevet FR 2669022 décrit l'utilisation d'un catalyseur à base de nickel et de chrome supportés sur AlF₃ ou alumine fluorée pour la fluoration spécifique du F133a (CF₃-CH₂Cl) en F134a (CF₃-CH₂F), ce catalyseur permettant d'obtenir de très bonnes sélectivités en F134a.

Vu l'intérêt des composés F124 et F125 comme substituts aux CFC, leur fabrication industrielle nécessite un procédé particulièrement performant, c'est-à-dire permettant d'obtenir :
- une très grande sélectivité en F124 + F125
- une productivité élevée en F124 et/ou F125
- une grande souplesse pour orienter à volonté la fabrication vers la production majoritaire de F124 ou vers celle de F125, tout en minimisant la formation de sous-produits.

Un procédé permettant d'atteindre cet objectif a été décrit dans la demande de brevet FR 2 661 906 qui, pour la fluoration du F123, préconise un catalyseur d'oxyde de chrome supporté sur charbon actif.

Il a maintenant été trouvé que cet objectif peut également être atteint en utilisant le catalyseur mixte Ni-Cr selon la demande de brevet FR 2 669 022 relative à la fluoration du F133a en F134a. Ce type de catalyseur permet d'obtenir une sélectivité totale en F124+F125 d'environ 90 % ou plus.

L'invention a donc pour objet un procédé continu de préparation du F124 et/ou du F125 par fluoration catalytique en phase gazeuse d'au moins un pentahalogénoéthane de formule C₂HX₂₋ₙF₃₊ₙ dans laquelle X désigne un atome de chlore ou de brome et n le nombre 0 ou 1, au moyen d'acide fluorhydrique, caractérisé en ce que l'on utilise un catalyseur mixte composé d'oxydes, halogénures et/ou oxyhalogénures de nickel et de chrome déposés sur un support constitué de fluorure d'aluminium ou d'un mélange de fluorure d'aluminium et d'alumine.

Le pentahalogénoéthane de départ est de préférence le F123, seul ou en mélange avec du F123a (1,2-dichloro-1,1,2-trifluoroéthane). Cependant, pour la fabrication préférentielle du F125, on peut aussi utiliser comme produit de départ le F124 lui-même, son isomère F124a (1-chloro-1,1,2,2-tétrafluoroéthane), le F123b (1,1-dichloro-1,2,2-trifluoroéthane) ou un mélange de ces composés.

Bien qu'on préfère partir des pentahalogénoéthanes chlorés C₂HCl₂₋ₙF₃₊ₙ, le procédé selon l'invention peut s'appliquer à leurs homologues bromés tels que, par exemple, le 1-bromo-1-chloro-2,2,2-trifluoroéthane (CF₃CHBrCl), le 1,1-dibromo-2,2,2-trifluoroéthane (CF₃CHBr₂), le 1-bromo-2-chloro-1,1,2-trifluoroéthane (CF₂BrCHClF) ou 1,1-dibromo-1,2,2-trifluoroéthane (CFBr₂CHF₂).

Le catalyseur à utiliser conformément à la présente invention peut être préparé de façon connue en soi à partir d'une alumine activée. Celle-ci peut dans une première étape être transformée en fluorure d'aluminium ou en mélange de fluorure d'aluminium et d'alumine, par fluoration à l'aide d'air (ou d'un inerte tel l'azote) et d'acide fluorhydrique, le taux de transformation de l'alumine en fluorure d'aluminium dépendant essentiellement de la température à laquelle est effectuée la fluoration de l'alumine (en général entre 200 et 450°C, de préférence entre 250 et 400°C). Le support est ensuite imprégné à l'aide de solutions aqueuses de sels de chrome et de nickel ou à l'aide de solutions aqueuses d'acide chromique, de sel de nickel et d'un réducteur du chrome comme le méthanol.

Lorsque l'on utilise l'acide chromique (CrO₃) comme précurseur du chrome, on peut réduire ce chrome par tout moyen connu de l'homme de l'art (réducteur chimique, réduction thermique ...), sous réserve que la technique utilisée ne nuise pas aux propriétés du catalyseur et donc à son activité . Le réducteur chimique préféré est le méthanol.

Comme sels de chrome et de nickel, on préfère utiliser les chlorures, mais on peut aussi employer d'autres sels tels que, par exemple, les oxalates, formiates, acétates, nitrates et sulfates ou le bichromate de nickel pourvu que ces sels soient solubles dans la quantité d'eau susceptible d'être absorbée par le support.

Le catalyseur utilisé dans le procédé selon l'invention peut aussi être préparé par imprégnation directe de l'alumine par des solutions des composés de chrome et de nickel ci-dessus mentionnées. Dans ce cas, la transformation d'au moins une partie (70 % ou plus) de l'alumine en fluorure d'aluminium s'effectue lors de l'étape d'activation du catalyseur.

Les alumines activées à utiliser pour la préparation du catalyseur selon la présente invention sont des produits bien connus, disponibles dans le commerce. Elles sont généralement préparées par calcination d'hydrates d'alumine à une température comprise entre 300 et 800°C. Les alumines activées, utilisables dans le cadre de la présente invention, peuvent contenir des teneurs importantes (jusqu'à 1000 ppm) de sodium sans que cela nuise à l'activité catalytique.

Le catalyseur selon l'invention peut contenir en masse de 0,5 à 20 % de chrome et de 0,5 à 20 % de nickel et, de préférence, entre 2 et 10 % de chacun des métaux dans un rapport atomique nickel/chrome compris entre 0,5 et 5, de préférence voisin de 1.

Avant de pouvoir catalyser la réaction de fluoration du pentahalogénoéthane C₂HX₂₋ₙF₃₊ₙ, le catalyseur selon l'invention doit être conditionné, c'est-à-dire transformé en constituants actifs et stables (aux conditions réactionnelles) par une opération préalable dite d'activation.

Ce traitement peut être réalisé soit "in situ" (dans le réacteur de fluoration) ou bien dans un appareillage adéquat conçu pour résister aux conditions d'activation. Celle-ci comprend généralement les étapes suivantes :
- séchage à basse température (100 à 150°C, de préférence 110 à 130°C) en présence d'air ou d'azote,
- séchage à haute température (250 à 450°C, de préférence 300 à 350°C) sous azote ou sous air,
- fluoration à basse température (180 à 300°C, de préférence à 200°C environ) au moyen d'un mélange d'acide fluorhydrique et d'azote, en contrôlant la teneur en HF de façon que la température ne dépasse pas 300°C, et
- finition sous courant d'acide fluorhydrique pur ou dilué par de l'azote à une température pouvant aller jusqu'à 450°C.

Pendant cette opération, les précurseurs catalytiques (halogénures de nickel et de chrome, chromate, bichromate de nickel, oxyde de chrome) sont transformés en fluorures et/ou oxyfluorures correspondants, ce qui entraîne un dégagement d'eau et/ou d'acide chlorhydrique.

Cette activation contribue également à accroître la fluoration de l'alumine lorsque l'on a réalisé l'imprégnation sur un support déjà partiellement fluoré, ou lorsque l'on imprègne directement l'alumine, à la fluoration de cette dernière. Dans ce dernier cas, il est nécessaire de contrôler parfaitement la température (la fluoration de l'alumine est très exothermique) si l'on ne veut pas nuire aux caractéristiques physiques du catalyseur; par ailleurs, les quantités d'eau générées sont nettement plus importantes.

L'analyse chimique des éléments (chrome, nickel, fluor, aluminium, oxygène), après activation, permet de vérifier la composition minérale du catalyseur selon l'invention.

Conformément au procédé selon l'invention, la réaction de fluoration du pentahalogénoéthane par HF peut être conduite à une température allant de 250 à 470°C et, plus particulièrement, à une température comprise entre 280 et 410°C. Cependant, si l'on désire orienter la réaction vers la synthèse préférentielle du F124, on travaillera plutôt à une température située dans la partie basse (300-330°C) du domaine précité; inversement, une température plus élevée favorise la synthèse du F125.

Le temps de contact pour la réaction selon l'invention peut être compris entre 3 et 100 secondes et, plus particulièrement, entre 5 et 30 secondes. Cependant, afin d'obtenir un bon compromis entre le taux de conversion du F123 et des productivités élevées de F124 et/ou F125, la meilleure plage est de 7 à 15 secondes.

Le rapport molaire HF/pentahalogénoéthane peut aller de 1/1 à 20/1 et préférentiellement de 2/1 à 9/1. Là encore, le taux de conversion du F123 et la distribution des produits formés varient avec le rapport molaire choisi, une augmentation du rapport molaire conduisant à une amélioration du taux de conversion du F123 et à un déplacement des produits formés vers des composés plus fluorés (F125). Il faut toutefois noter qu'un rapport molaire faible (inférieur à 2) augmente la formation de produits non recyclables (perhalogénoéthanes et tétrahalogénoéthanes).

La réaction de fluoration selon l'invention peut être conduite dans un réacteur de fluoration en phase gazeuse en lit fixe ou en lit fluide. Les matériaux utilisés pour la construction de l'installation doivent être compatibles avec la présence d'hydracides tels que HCl et HF; ils peuvent être choisis en "Hastelloy" ou en "Inconel" qui sont résistants aux milieux corrosifs contenant ces hydracides.

La réaction de fluoration selon l'invention peut être menée à la pression atmosphérique ou à une pression supérieure à cette dernière. Pour des raisons pratiques, on opérera généralement dans une zone allant de 0 à 25 bars relatifs.

Dans des conditions opératoires susceptibles d'encrasser le catalyseur, il peut être judicieux d'introduire avec les réactifs, de l'oxygène à faible teneur. Cette teneur peut varier selon les conditions opératoires entre 0,02 et 5 % par rapport aux réactifs organiques (pourcentage molaire). Cet oxydant a pour but de réagir avec les "lourds" qui sont à l'origine de l'encrassement du catalyseur.

La mise en oeuvre du procédé selon l'invention permet d'obtenir les deux pentahalogénoéthanes désirés F124 et F125 avec une excellente sélectivité (égale ou supérieure à environ 90 %), la proportion d'isomères a et de produits non recyclables (perhalogénoéthanes et tétrahalogénoéthanes) étant très faible. Le procédé selon l'invention présente également une grande souplesse; à partir de F123, le rapport molaire F124/F125 dans le produit obtenu peut varier de 10/1 à 1/10 selon les conditions opératoires. D'autre part, la possibilité d'opérer avec des rapports molaires et des temps de contact faibles permet d'obtenir une bonne productivité, tout en ayant un taux satisfaisant de transformation du F123.

Les produits sous-fluorés éventuellement formés (F122: 0-0,2 % et F1111: 0,1-1,2 %) peuvent être recyclés avec les F123 et F123a non transformés. Si on le désire, les composés F124 et F124a peuvent également être recyclés au réacteur pour augmenter la productivité en F125.

L'exemple suivant illustre l'invention sans la limiter.

### EXEMPLE

### A - PREPARATION ET ACTIVATION DU CATALYSEUR

Dans un évaporateur rotatif, on place 250 ml d'un support contenant en poids 73 % de fluorure d'aluminium et 27 % d'alumine, obtenu dans une étape précédente par fluoration d'alumine GRACE HSA en lit fluidisé vers 300°C à l'aide d'air et d'acide fluorhydrique (concentration volumique de 5 à 10 % de cet acide dans l'air). L'alumine GRACE HSA de départ présente les caractéristiques physicochimiques suivantes :
- forme : billes de 1-2 mm de diamètre
- surface BET: 220m²/g
- volume poreux : 1,2 cm³/g (pour les rayons de pores compris entre 4 nm et 63 µm)
- teneur en sodium : 600 ppm

On prépare par ailleurs deux solutions aqueuses séparées :

| **(a)** solution chromique additionnée de chlorure de nickel contenant : | |
|---|---|
| - anhydride chromique | 25 g |
| - chlorure de nickel hexahydraté | 58 g |
| - eau | 40 g |

| **(b)** Solution méthanolique contenant : | |
|---|---|
| - méthanol | 35 g |
| - eau | 30 g |

Le mélange de ces deux solutions est ensuite introduit, à température ambiante sous pression atmosphérique et en 45 minutes environ, sur le support en agitation. Le catalyseur est alors séché sous courant d'azote, en lit fluidisé, vers 110°C pendant 4 heures.

On charge 100 ml (77,5 g) de catalyseur sec dans un réacteur tubulaire en INCONEL de diamètre intérieur 27 mm et l'on monte la température à 120°C sous courant d'azote, à pression atmosphérique. On maintient ce traitement pendant une dizaine d'heures puis on remplace progressivement l'azote par de l'acide fluorhydrique en veillant à ce que l'augmentation de température n'excède pas 95°C et, lorsque l'on atteint un ratio molaire HF/N₂ de 50/50, on élève la température à 300°C.

Après disparition des pics d'exothermie, on monte la température à 350°C sous courant d'acide fluorhydrique pur (1 mole/heure) pendant 6 heures.

Le catalyseur est finalement balayé sous courant d'azote avant de démarrer le test catalytique. Les caractéristiques du catalyseur ainsi séché et activé sont les suivantes :

| - composition chimique (pondérale) | |
|---|---|
| . fluor | 58,5 % |
| . aluminium | 25,1 % |
| . nickel | 6,8 % |
| . chrome | 5,6 % |
| . oxygène | 4 % |

| - propriétés physiques : | |
|---|---|
| . surface BET | 15,1 m²/g |
| . volume des pores d'un rayon compris entre 4 nm et 63 µm | 0,382 cm³/g |
| . surface des pores de rayon supérieur à 4 nm | 18 m²/g |

### B - FLUORATION DU F123

Les performances du catalyseur dans la fluoration du F123 ont été testées à pression atmosphérique, sans addition d'oxygène, dans les conditions opératoires et avec les résultats rassemblés dans le tableau 1 suivant.

Le F123 utilisé est un produit brut constitué essentiellement de F123 (96,1 %), les autres composés étant le F123a (3,6 %), le F123b (moins de 0,1 %) et le F113 (environ 0,1 %).

**TABLEAU 1**

| **TEST N°** | **B1** | **B2** |
|---|---|---|
| ***Conditions opératoires*** : | | |
| - Température (°C) | 350 | 300 |
| - Rapport molaire HF/F123 | 5,5 | 2,9 |
| - Temps de contact (s) | 9,8 | 10,9 |
| - Age du catalyseur (h) | 41 | 81 |

| ***Résultats*** : | | |
|---|---|---|
| - Taux de transformation global du F123 (%) | 81,6 | 44,2 |

| - Sélectivité (% molaire) en : | | |
|---|---|---|
| F125 | 56,7 | 10,7 |
| F124 | 42,2 | 87,1 |
| F124a | 0,1 | 0,2 |
| F123a | 0,1 | 0,2 |
| F133a | 0,3 | traces |
| F115 | 0,2 | 0 |
| F114 + F 114a (C₂F₄Cl₂) | 0,3 | 0,1 |
| F1111(CFCl = CCl₂) | 0,1 | traces |

### C - FLUORATION DU F124

Les performances du même catalyseur dans la fluoration du F124 ont été testées à pression atmosphérique, sans addition d'oxygène, dans les conditions opératoires et avec les résultats rassemblés dans le tableau 2 suivant. Le F124 de départ contient 0,9 % d'isomère F124a.

**TABLEAU 2**

| **TEST N°** | **C1** | **C2** |
|---|---|---|
| ***Conditions opératoires*** : | | |
| - Température (°C) | 350 | 300 |
| - Rapport molaire HF/F124 | 3 | 2,9 |
| - Temps de contact (s) | 13,1 | 10,2 |
| - Age du catalyseur (h) | 30 | 75 |

| ***Résultats*** : | | |
|---|---|---|
| - Taux de transformation global du F124 (%) | 86,7 | 89,7 |

| - Sélectivité (% molaire) en : | | |
|---|---|---|
| F125 | 91,1 | 89,8 |
| F124a | 0,2 | 0,3 |
| F123 | 7,4 | 5,4 |
| F123a | traces | traces |
| F133a | 0,5 | 1,3 |
| F115 | 0,3 | 1,3 |
| F114 + F114a (C₂F₄Cl₂) | 0,3 | 0,6 |
| F1111(CFCl = CCl₂) | 0,1 | 0,3 |
| F1110 (CCl₂ = CCl₂) | 0,1 | 0,3 |

## Revendications

1. Procédé de préparation du 1,1,1,2-tétrafluoro-2-chloroéthane et/ou du pentafluoroéthane par fluoration catalytique en phase gazeuse d'au moins un pentahalogénoéthane de formule C₂HX₂₋ₙF₃₊ₙ dans laquelle X désigne un atome de chlore ou de brome et n le nombre 0 ou 1, au moyen d'acide fluorhydrique, caractérisé en ce que l'on utilise un catalyseur mixte composé d'oxydes, halogénures et/ou oxyhalogénures de nickel et de chrome déposés sur un support constitué de fluorure d'aluminium ou d'un mélange de fluorure d'aluminium et d'alumine.

2. Procédé selon la revendication 1, caractérisé en ce que la teneur pondérale du catalyseur en nickel et en chrome est comprise entre 0,5 et 20 % pour chaque métal, le rapport atomique nickel/chrome étant compris entre 0,5 et 5, de préférence voisin de 1.

3. Procédé selon la revendication 2, caractérisé en ce que la teneur est comprise entre 2 et 10 % pour chaque métal.

4. Procédé selon l'une des revendications 1 à 3, dans lequel l'acide fluorhydrique et le(s) pentahalogénoéthane(s) sont mis en contact dans un rapport molaire HF/pentahalogénoéthane(s) compris entre 1/1 et 20/1, pendant un temps compris entre 3 et 100 secondes et à une température comprise entre 250 et 470°C.

5. Procédé selon la revendication 4, dans lequel le rapport molaire est compris entre 2/1 et 9/1, le temps de contact est compris entre 5 et 30 secondes, de préférence entre 7 et 15 secondes, et la température est comprise entre 280 et 410°C.

6. Procédé selon l'une des revendications 1 à 5, dans lequel le pentahalogénoéthane de départ est le 1,1,1-trifluoro-2,2-dichloroéthane et/ou le 1,1,1,2-tétrafluoro-2-chloroéthane.

7. Procédé selon l'une des revendications 1 à 6, dans lequel on travaille à une pression allant de 0 à 25 bars relatifs, de préférence à la pression atmosphérique.

8. Procédé selon l'une des revendications 1 à 7, dans lequel on opère en présence d'oxygène.

## Claims

1. Process for the preparation of 1,1,1,2-tetrafluoro-2-chloroethane and/or of pentafluoroethane by gas phase catalytic fluorination of at least one pentahaloethane of formula C₂HX₂₋ₙF₃₊ₙ in which X denotes a chlorine or bromine atom and n the number 0 or 1 by means of hydrofluoric acid, characterized in that use is made of a mixed catalyst composed of oxides, halides and/or oxyhalides of nickel and chromium deposited on a support consisting of aluminium fluoride or of a mixture of aluminium fluoride and alumina.

2. Process according to Claim 1, characterized in that the content, by weight, of nickel and chromium in the catalyst is between 0.5 and 20 % for each metal, the nickel/chromium atomic ratio being between 0.5 and 5, preferably in the region of 1.

3. Process according to Claim 2, characterized in that the content is between 2 and 10 % for each metal.

4. Process according to one of Claims 1 to 3, in which the hydrofluoric acid and the pentahaloethane(s) are brought into contact in an HF/pentahaloethane(s) molar ratio between 1/1 and 20/1, for a time between 3 and 100 seconds and at a temperature between 250 and 470°C.

5. Process according to Claim 4, in which the molar ratio is between 2/1 and 9/1, the contact time is between 5 and 30 seconds, preferably between 7 and 15 seconds, and the temperature is between 280 and 410°C.

6. Process according to one of Claims 1 to 5, in which the starting pentahaloethane is 1,1,1-trifluoro-2,2-dichloroethane and/or 1,1,1,2-tetrafluoro-2-chloroethane.

7. Process according to one of Claims 1 to 6, in which the process is carried out at a pressure ranging from 0 to 25 bar relative, preferably at atmospheric pressure.

8. Process according to one of Claims 1 to 7, in which the reaction is carried out in the presence of oxygen.

## Patentansprüche

1. Verfahren zur Herstellung von 1,1,1,2-Tetrafluor-2-chlorethan und/oder Pentafluorethan durch katalytische Fluorierung in Gasphase mindestens eines Pentahalogenethans der Formel C₂HX₂₋ₙF₃₊ₙ, in der X ein Chlor- oder Bromatom und n die Zahlen 0 oder 1 bezeichnet, mittels Fluorwasserstoff, dadurch gekennzeichnet, daß man einen Mischkatalysator aus Oxiden, Halogeniden und/oder Oxyhalogeniden des Nickels und des Chroms verwendet, die aufgebracht sind auf einem Träger aus Aluminiumfluorid oder einer Mischung aus Aluminiumfluorid und Aluminiumoxid.

2. Verfahren nach Anspruch 1, dadurch gekennzeichnet, daß der Gewichtsgehalt des Katalysators an Nickel und Chrom zwischen 0,5 und 20 % für jedes Material liegt, wobei das Nickel/Chrom-Atomverhältnis zwischen 0,5 und 5, vorzugsweise bei etwa 1, liegt.

3. Verfahren nach Anspruch 2, dadurch gekennzeichnet, daß der Gehalt zwischen 2 und 10 % fuhr jedes Metall liegt.

4. Verfahren nach einem der Ansprüche 1 bis 3, bei dem der Fluorwasserstoff und das oder die Pentahalogenethane in einem HF/Pentahalogenethan(e)-Molverhältnis zwischen 1/1 und 20/1 während einer Zeit zwischen 3 und 100 Sekunden und bei einer Temperatur zwischen 250 und 470 °C in Kontakt gebracht werden.

5. Verfahren nach Anspruch 4, bei dem das Molverhältnis zwischen 2/1 und 9/1, die Kontaktzeit zwischen 5 und 30 Sekunden, vorzugsweise zwischen 7 und 15 Sekunden, und die Temperatur zwischen 280 und 410 °C liegt.

6. Verfahren nach einem der Ansprüche 1 bis 5, bei dem das Ausgangspentahalogenethan 1,1,1-Trifluor-2,2-dichlorethan und/oder 1,1,1,2-Tetrafluor-2-chlorethan ist.

7. Verfahren nach einem der Ansprüche 1 bis 6, bei dem man bei einem Druck von 0 bis 25 bar relativ, vorzugsweise bei Atmosphärendruck, verfährt.

8. Verfahren nach einem der Ansprüche 1 bis 7, bei dem man in Gegenwart von Sauerstoff verfährt.
